(19) **Europäisches Patentamt**
**European Patent Office**
**Office européen des brevets**

(11) **EP 4 151 203 B1**

(12) **FASCICULE DE BREVET EUROPEEN**

(45) Date de publication et mention de la délivrance du brevet:
**02.04.2025   Bulletin 2025/14**

(21) Numéro de dépôt: **22194963.9**

(22) Date de dépôt: **09.09.2022**

(51) Classification Internationale des Brevets (IPC):
**A61K 8/9789** (2017.01)   **A61Q 17/04** (2006.01)
**A61Q 19/04** (2006.01)   **A61Q 19/08** (2006.01)
**A61Q 19/06** (2006.01)

(52) Classification Coopérative des Brevets (CPC):
**A61Q 19/08; A61K 8/9789; A61Q 17/04; A61Q 19/04; A61Q 19/06;** A61K 2800/80

(54) **EXTRAIT DE TOURTEAU DE COLZA, PROCÉDÉ DE PRÉPARATION ET UTILISATION EN COSMÉTIQUE NOTAMMENT EN TANT QU AGENT PROPIGMENTANT, AMINCISSANT**

RAPSKUCHENEXTRAKT, VERFAHREN ZUR HERSTELLUNG UND VERWENDUNG IN DER KOSMETIK INSBESONDERE ALS PROPIGMENTIERENDES, SCHLANKMACHENDES MITTEL

RAPESEED MEAL EXTRACT, METHOD FOR PREPARING SAME AND USE IN COSMETICS, IN PARTICULAR AS A PRO-PIGMENTING, SLIMMING AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:   **20.09.2021   FR 2109860**

(43) Date de publication de la demande:
**22.03.2023   Bulletin 2023/12**

(73) Titulaire: **Lyster**
**86360 Chasseneuil-du-Poitou (FR)**

(72) Inventeurs:
• **BOURGETEAU, Vincent**
**69007 LYON (FR)**
• **PIGNOLET, François**
**69007 LYON (FR)**

(74) Mandataire: **Oak & Fox**
**94, rue La Fayette / Esc. D**
**75010 Paris (FR)**

(56) Documents cités:
**FR-A1- 2 904 555**

• KIM SUN MI ET AL: "A Study on Skin Care Effects of Rapeseed Meal Extract", vol. 28, no. 3, 27 June 2013 (2013-06-27), KR, pages 177 - 184, XP055928462, ISSN: 1225-7117, Retrieved from the Internet <URL:http://dx.doi.org/10.7841/ksbbj.2013.28.3.177> [retrieved on 20220607], DOI: 10.7841/ksbbj.2013.28.3.177
• GEORGIEV RADOSLAV ET AL: "Phytochemical Profile and Bioactivity of Industrial Rapeseed Meal Ethanol-Wash Solutes", WASTE AND BIOMASS VALORIZATION, SPRINGER NETHERLANDS, NL, vol. 12, no. 9, 13 February 2021 (2021-02-13), pages 5051 - 5063, XP037521952, ISSN: 1877-2641, [retrieved on 20210213], DOI: 10.1007/S12649-021-01373-6
• JANG BOO-SIK ET AL: "Preparation and availability analysis of glycoprotein from canola meal", JOURNAL OF FOOD SCIENCE AND TECHNOLOGY, vol. 58, no. 1, January 2021 (2021-01-01), pages 377 - 382, XP037339439, ISSN: 0022-1155, DOI: 10.1007/S13197-020-04586-0

**Description**

[La présente invention se rapporte au domaine de la valorisation du tourteau de colza. Plus spécifiquement l'invention concerne un extrait de tourteau de colza ainsi que son utilisation en cosmétique en application sur la peau notamment en tant qu'agent propigmentant, amincissant, relaxant.

La peau est la principale barrière de protection du corps humain à l'égard des agressions extérieures telles que la pollution atmosphérique, les variations climatiques et le rayonnement UV.

La peau est constituée de trois couches : l'épiderme, le derme et l'hypoderme. L'épiderme, qui est en contact avec l'extérieur, est plus particulièrement concerné par les agressions extérieures. L'épiderme est recouvert par un film hydrolipidique et est composé de quatre couches : la couche cornée, la couche granuleuse, la couche épineuse et la couche basale.

La peau, et en particulier l'épiderme, est ainsi constamment soumise à des stress qui notamment engendrent son vieillissement.

Or, l'aspect de la peau a, de tout temps, revêtu une importance fondamentale : en particulier le maintien d'une peau d'aspect jeune et en bonne santé sont des qualités constamment recherchées.

Ainsi il demeure toujours un besoin de compositions cosmétiques permettant de conférer à la peau, notamment du visage, un aspect bonne mine, aminci, relaxé.

La mélanine est le pigment responsable de la coloration de la peau, dans les mélanocytes situés à la surface de la peau. Le mécanisme de formation de la pigmentation de la peau, c'est-à-dire de la mélanine, est particulièrement complexe et fait intervenir schématiquement les principales étapes suivantes, au niveau des mélanocytes de la couche basale :

**Tyrosine→DOPA→Dopaquinone →leucodopachrome → dopachrome → mélanine**

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase.

FR 2 904 555 A1 décrit l'utilisation dans une composition cosmétique ou pour la préparation d'une composition pharmaceutique, d'une quantité efficace d'extrait de tourteau de colza (hydrolysat) en tant qu'actif propigmentant, autobronzant. Mais de nouvelles compositions cosmétiques destinées à être appliquées sur la peau et qui présentent des propriétés propigmentantes sont toujours recherchées.

Les inventeurs ont montré qu'une solution à ce problème technique de l'obtention de nouvelles compositions cosmétiques pouvait être apportée au moyen d'un extrait particulier de tourteau de colza.

Le colza est une plante annuelle à fleurs jaunes de la famille des Brassicacées. Le colza est essentiellement cultivé pour l'alimentation animale et la production d'huile alimentaire. Depuis quelques décennies, le colza est également utilisé, en quantité croissante, en tant que biocarburant.

L'huile de colza est obtenue par pressage, puis raffinage plus ou moins poussé du colza. La production d'huile de colza génère une grande quantité de sous-produits solides appelés tourteaux de colza.

Le tourteau de colza constitue une source de protéines et, à ce titre, il est utilisé dans des formulations pour l'alimentation animale plus particulièrement pour les bovins, porcins et ovins.

Il demeure cependant toujours un besoin de trouver de nouvelles voies de valorisation du tourteau de colza à haute valeur ajoutée.

De manière surprenante et avantageuse les inventeurs ont mis en évidence un extrait particulier de tourteau de colza obtenu par un procédé d'extraction à l'éthanol particulièrement innovant dans le traitement des tourteaux de colza, et présentant des effets avantageux en cosmétique en application sur la peau, plus particulièrement en tant qu'agent propigmentant, et amincissant.

La présente invention vise un extrait de tourteau de colza susceptible d'être obtenu par un procédé de préparation comprenant au moins les étapes suivantes, dans cet ordre :

- fournir des graines de colza,
- presser les graines de colza et récupérer le tourteau,
- broyer le tourteau pour obtenir une taille moyenne des particules inférieure ou égale 5 mm,
- procéder à l'extraction du tourteau de colza au moyen d'éthanol à 96°,
- filtrer,
- récupérer le filtrat,
- évaporer le solvant,
- récupérer l'extrait obtenu.

L'extrait de tourteau de colza selon l'invention contient une matière première organique (MO) riche en composés azotés.

L'extrait selon l'invention contient plusieurs types d'actifs d'intérêt en cosmétique c'est-à-dire à la fois des corps gras aminés : amides gras et/ou céramides, ainsi que des protéines et des polyphénols.

La présente invention vise encore une composition non thérapeutique, cosmétique ou alimentaire, comprenant au moins un extrait selon l'invention, de préférence en une teneur allant de 0,001 à 30 % en poids et de préférence de 0,1 à 20 % en poids par rapport au poids total de la composition.

La composition cosmétique selon l'invention est une composition propigmentante, autobronzante. La composition présente aussi des propriétés amincissantes notamment elle permet d'affiner le contour du visage. Elle permet également de conférer à la peau un effet apaisant, relaxant, anti-stress.

Ces compositions cosmétiques sont en outre stables, non irritantes, non toxiques, non allergisantes pour la peau.

D'autres aspects, avantages, propriétés de la présente invention sont présentés dans la description et les exemples qui suivent.

**Définitions**

Dans le présent texte, sauf indications contraires spécifiques, les pourcentages sont exprimés en poids d'une composition de référence.

Dans le présent texte, les intervalles sont définis de façon abrégée afin d'éviter de décrire chacune des valeurs et toutes les valeurs de cet intervalle, cependant toute valeur appropriée dans l'intervalle peut être choisie comme la valeur supérieure, la valeur inférieure ou les valeurs terminales de l'intervalle. Par exemple, un intervalle de 0,1 à 1,0 représente les valeurs terminales de 0,1 et 1,0, ainsi que les valeurs intermédiaires de 0,2 ; 0,3 ; 0,4 ; 0,5 ; 0,6 ; 0,7 ; 0,8 ; 0,9 et tous les intervalles intermédiaires compris dans 0,1 à 1,0, tels que 0,2 à 0,5 ; 0,2 à 0,8 ; 0,7 à 1,0... Un intervalle défini comme étant « compris entre la valeur A et la valeur B » inclut les valeurs A et B et est donc équivalent à un intervalle « allant de la valeur A à la valeur B ». En outre, l'expression « au moins » comprend la valeur énoncée après, par exemple, « au moins 5 % » doit être compris comme comprenant également « 5 % ». L'expression « un maximum de » comprend la valeur énoncée après, par exemple, « un maximum de 5 % » doit être compris comme comprenant également « 5 % ».

En outre, dans le présent texte, les valeurs mesurables, telles qu'une quantité, doivent être comprises comme comprenant les écarts types qui peuvent être facilement déterminés par l'homme du métier dans le domaine technique de référence. De préférence, ces valeurs sont destinées à comprendre des variations de $\pm$ 5 %.

Dans le présent texte, la forme au singulier d'un mot comprend le pluriel et inversement, sauf si le contexte indique clairement le contraire. Ainsi, les références « un », « une » et « le/la » comprennent généralement les pluriels des termes respectifs. Par exemple, une référence à un « procédé » ou une « composition » comprend une pluralité de ces « procédés » ou « compositions ». En particulier, dans le présent texte, les termes « tourteau de colza » et « tourteaux de colza » sont utilisés de manière indifférentiée.

Par « composé volatil » au sens de la présente invention, on entend un composé dont la pression de vapeur à 20°C (293,15 K) est supérieure ou égale à 0,01 kPa. Cette définition est en conformité avec la directive du Conseil Européen 1999/13/CE.

Par « peau » on entend l'épiderme du visage ou du corps et en particulier du visage, du cou, du décolleté et des mains.

Par « prévenir » ou « prévention » on entend le fait de diminuer, au moins en partie, le risque de survenue d'un effet.

Les compositions selon la présente invention sont des compositions alimentaires ou cosmétiques, non thérapeutiques. Les compositions cosmétiques sont destinées à être appliquées sur les peaux saines, par simple massage pour traiter l'épiderme. Elles sont conformes au règlement CE 1223/2009.

**Extrait de tourteau de colza**

L'extrait selon la présente invention est préparé à partir de tourteaux de colza, plus particulièrement à partir de tourteaux de graines de colza (*Brassica napus*).

Il est connu que les graines de *Brassica napus* sont utilisées pour l'extraction de leur huile, utile pour l'autoconsommation ou en soins médicinaux traditionnels divers. Le tourteau obtenu après avoir déshuilé la graine est un déchet actuellement utilisé pour la nourriture notamment des animaux.

Selon un mode de réalisation préférentiel, l'extrait selon l'invention est obtenu à partir du tourteau des graines non décortiquées de *Brassica napus.*

Selon la présente invention, les tourteaux de colza sont récupérés après l'extraction de l'huile de colza par pressage puis extraits à l'éthanol. Le pressage est avantageusement effectué au moyen d'une presse mécanique, sans solvant.

L'extrait de tourteau de colza selon l'invention présente un taux de matières sèches proche de 100% en poids par rapport au poids total de l'extrait.

Après extraction du solvant (éthanol) notamment par évaporation sous vide à 45°C, l'extrait contient de la Matière Organique (MO) issue du tourteau de colza.

Après élimination du solvant, le taux de matière organique (MO) va de 5 à 10%, préférentiellement de 7 à 8% en poids par rapport au poids de tourteau initial.

Selon une première variante, l'extrait de tourteau de colza selon la présente invention comprend de 1 à 5% de préférence 2 % en poids de protéines par rapport au poids total de l'extrait.

Selon une deuxième variante, l'extrait de tourteau de colza selon la présente invention présente une teneur en polyphénols allant de 100 à 150 ppm, et de préférence 135 ppm.

Selon une troisième variante, l'extrait de tourteau de colza selon la présente invention comprend de 50 à 75% en poids d'huile de colza et de 25 à 50% en poids de composés volatils par rapport au poids total de l'extrait.

L'extrait de tourteau de colza selon l'invention comprend une phase des composés volatils particulière comprenant des céramides et amides gras en $C_8$-$C_{24}$, ainsi que des tocophérols.

Ainsi, de préférence, l'extrait de tourteau de colza selon l'invention comprend de 0,5 à 1,25 %, de préférence 0,9% en poids de composés choisis parmi les céramides et les amides gras en $C_8$-$C_{24}$ par rapport au poids total de l'extrait.

De préférence, l'extrait de tourteau de colza selon l'invention comprend de 0,17 % à 0,33 % de préférence 0,25 % en poids de gamma-tocophérols par rapport au poids total de l'extrait et de 0,14 % à 0,28 % de préférence 0,20 % en poids d'alpha-tocophérols par rapport au poids total de l'extrait.

**Procédé de préparation de l'extrait**

La présente invention a aussi pour objet un procédé de préparation de de l'extrait de tourteau de colza comprenant au moins les étapes suivantes, dans cet ordre :

- fournir des graines de colza,
- presser les graines de colza et récupérer le tourteau,
- broyer le tourteau pour obtenir une taille moyenne des particules inférieure ou égale 5 mm,
- procéder à l'extraction du tourteau de colza au moyen d'éthanol à 96°,
- filtrer,
- récupérer le filtrat,
- évaporer le solvant,
- récupérer l'extrait obtenu.

Ainsi, les premières étapes du procédé de préparation de l'extrait de tourteau de colza comprennent :

- fournir des graines de colza ;
- presser les graines de colza et récupérer le tourteau ;
- broyer le tourteau issu de la délipidation mécanique des graines.

De préférence, les graines de colza destinées à être utilisées dans le procédé selon l'invention sont non décortiquées.

De préférence elles sont séchées ou laissées sécher de manière à obtenir un taux d'humidité inférieur à 7%.

Puis les graines sont pressées de manière à séparer l'huile du reste de la graine et à obtenir le tourteau. Généralement le tourteau comprend moins de 5% en poids d'huile dite « huile résiduelle ».

Selon une variante préférée, le tourteau est ensuite broyé mécaniquement avec tout type de broyeurs mécaniques tels que des systèmes à marteaux, à fléau, à couteaux, à concassage/déchiquetage, à billes ou boulets ou à pilon, mais aussi avec tout type de cryobroyeur.

Généralement, le tourteau est d'abord broyé à sec, puis broyé dans son solvant.

Le broyage du tourteau de colza permet avantageusement d'obtenir la taille de particule appropriée. Par « taille », on entend la longueur de la plus grande dimension des particules. Généralement, les particules de tourteau de colza présentent une taille inférieure ou égale à 5 mm, de préférence inférieure ou égale à 3 mm, et de manière préférée allant de 0,5 à 2 mm.

Le tourteau, avantageusement broyé, est ensuite placé dans une solution d'éthanol.

De préférence, l'extraction est effectuée pendant une durée allant de 1,5 à 4 heures, de préférence 2 heures, à une température allant de 12 à 45°C, de préférence à température ambiante c'est-à-dire entre 15 et 25 °C.

En outre, lors de l'extraction, le ratio masse de tourteau de colza / masse d'éthanol est compris entre 1/1 et 1/4 et de manière préférée d'environ 1/2.

L'extraction à l'éthanol est avantageusement effectuée sous agitation, généralement avec une hélice, permettant ainsi une dispersion et une homogénéisation du solide dans le liquide, améliorant ainsi la surface d'échange globale et par conséquent l'extraction.

A l'issue de cette étape le mélange est filtré, généralement à un seuil de filtration de 10 microns, et le filtrat est récupéré puis le solvant est évaporé.

L'évaporation est de préférence effectuée sous vide.

Le procédé selon l'invention peut également comprendre des étapes de purification dont la mise en œuvre relève des compétences de l'homme du métier.

Avantageusement, l'extrait obtenu a la capacité de se solubiliser dans l'éthanol 96° selon un rapport 1/1.

## Composition

Comme déjà mentionné, la présente invention vise également une composition non thérapeutique, cosmétique, comprenant l'extrait de tourteau de colza selon la présente invention.

Avantageusement la composition selon la présente invention comprend de 0,001 à 30 % en poids et de préférence de 0,1 à 20 % en poids d'extrait de tourteau de colza par rapport au poids total de la composition.

Avantageusement la composition cosmétique selon la présente invention comprend, dans un milieu cosmétiquement acceptable, au moins un agent cosmétique choisi parmi les agents humectants, les épaississants, les agents de texture, les émulsifiants, les agents dispersants, les agents moussants, les émolients, les conservateurs, les colorants, les extraits de plantes, les fibres végétales, les minéraux, les agents correcteurs de pH, les principes actifs et les parfums.

Avantageusement la composition selon la présente invention comprend de 0,001 à 20 % en poids d'au moins un agent cosmétique par rapport au poids total de la composition.

Bien entendu, l'homme du métier veillera à choisir ces agents cosmétiques de manière à ne pas altérer les propriétés de la composition selon l'invention.

La composition selon l'invention peut se présenter sous toutes les formes galéniques normalement utilisées dans le domaine cosmétique, et notamment sous forme d'une poudre ou de pastille, en particulier une pastille de dentifrice, d'un syndet en particulier un shampooing solide, d'une solution aqueuse ou hydroalcoolique, éventuellement gélifiée, éventuellement moussante, d'une dispersion du type lotion éventuellement biphasée, d'une émulsion huile-dans-eau ou eau-dans-huile ou multiple (E/H/E ou H/E/H par exemple), d'un gel aqueux, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non ionique; de gel aqueux ou huileux. Ces compositions sont préparées selon les méthodes usuelles.

La composition selon l'invention peut constituer une composition de soin de la peau, et notamment une crème de nettoyage, de protection, de traitement ou de soin pour la bouche, le visage, pour les mains, pour les pieds, ou pour le corps, en particulier la composition peut être une crème de jour, crème de nuit, crème démaquillante, crème de fond de teint, crème antisolaire ; un fond de teint fluide, un lait de démaquillage, un lait corporel de protection ou de soin, un lait antisolaire; une lotion, gel ou mousse pour le soin de la peau, comme une lotion de nettoyage.

La composition cosmétique selon la présente invention peut être utilisée en application topique sur la peau à titre d'agent propigmentant de la peau, ainsi qu'à titre d'agent amincissant de la peau.

Sans vouloir être lié à une quelconque théorie, les inventeurs ont montré que la composition cosmétique selon la présente invention est un activateur /stimulateur de la synthèse de la mélanine dans les mélanocytes humains normaux qui sont les cellules pigmentaires situées dans l'épiderme.

Les inventeurs ont aussi montré que la composition cosmétique selon la présente invention permet une augmentation de l'activité lipolyse des adipocytes humains. La composition cosmétique selon l'invention présente par conséquent des propriétés amincissantes, raffermissantes, qui contribuent à conférer un effet anti-age.

Cette activité lipolyse est aussi présente par une action stimulante de la production de ZAG (zinc Alphaglycoprotéine) par les kératinocytes de l'épiderme, la ZAG étant un mediateur clé impliqué dans la lipolyse par l'activation des récepteurs béta adrénergiques.

Enfin, il a également été observé que l'utilisation des compositions selon l'invention présente une action relaxante, anti-stress.

La composition cosmétique selon l'invention est avantageusement destinée à être appliquée sur la peau.

## Utilisations

La présente invention vise aussi l'utilisation cosmétique, non-thérapeutique d'au moins un extrait selon l'invention ou d'au moins une composition cosmétique selon l'invention en tant qu'actif propigmentant, autobronzant.

La présente invention vise encore l'utilisation cosmétique, non-thérapeutique d'au moins un extrait selon l'invention ou d'au moins une composition cosmétique selon l'invention en tant qu'actif amincissant notamment pour affermir la peau du contour du visage.

Elle vise également un procédé de traitement cosmétique, non thérapeutique de la peau, comprenant au moins une étape d'application sur la peau d'au moins un extrait selon l'invention ou d'au moins une composition cosmétique selon l'invention.

Les exemples qui suivent visent à illustrer l'invention sans en limiter la portée.

## Exemples

### Exemple 1- Extrait de tourteau de colza

#### I. Préparation de l'extrait

Dans un réacteur de 3 litres équipé d'un mélangeur à hélice 4 lames (Agitateur IKA RW20), ont été introduits 850 mL d'éthanol à 96°et 300 g de tourteau de colza, commercialisé par Centre Ouest Céréales. Le tourteau de colza a été préalablement broyé avec un broyeur à couteaux (SM100 RETSCH) de manière à obtenir une taille de particules allant de 0,5 à 2 mm.

Le mélange est dans un premier temps broyé dans son solvant à l'aide d'un mixer plongeant à lames durant 5 minutes.

Le mélange est alors maintenu sous agitation pendant 2 heures à température de 25°C.

A l'issue de ces 2 heures, le mélange a été filtré sur filtre plissé.

526,44 g de filtrat ont été obtenu, soit un rendement de 53,26%.

Puis le filtrat a été placé dans un rotavapor où l'évaporation de l'éthanol (486,11 g) a été effectuée. Après évaporation de l'éthanol, 23,39 g d'extrait désolvaté ont été obtenus, soit un rendement de 7,80% par rapport au poids initial de tourteaux.

L'extrait obtenu comprend un taux de matières sèches d'environ 100% en poids.

Dans le présent texte, le calcul du taux d'humidité et du taux de matières sèches est déterminé selon le protocole suivant.

On pèse environ 0,3 g d'un échantillon du produit à tester dans une coupelle en verre, la masse exacte est nommée Mav, puis on le place dans une étuve pendant 1 heure à 100°C. On pèse à nouveau l'échantillon, la masse exacte est nommée Map.

Le taux d'humidité TH correspond à :

$$TH = (Mav - Map)/ Mav)*100.$$

On effectue la mesure sur 2 échantillons et on retient la moyenne.

Le taux de matières sèches MS correspond à :

$$MS = 100\text{-}TH.$$

#### II. Détermination de la composition de l'extrait

##### A. Analyse par chromatographique gazeuse par détection à ionisation de flamme

L'extrait désolvaté obtenu à l'exemple 1 a été analysé par chromatographique gazeuse par détection à ionisation de flamme (GC/FID).

Selon cette méthode, l'échantillon est préparé selon une méthode de micro-extraction en phase solide dite SPME qui utilise comme support solide de piégeage la fibre CAR/PDMS (Carboxen®/Polydimethylsiloxane fibers). Le piègeage a lieu pendant 30 minutes à 90°C. Ce piégeage permet de séparer les composés volatils des triglycérides.

La fibre a été mise en saturation par ajout progressif de l'extrait de l'exemple 1. La fraction volatile est calculée selon :

Masse des composés volatils (MV)= Masse saturée de la fibre - masse initiale de la fibre

Le pourcentage de composés volatils est obtenu selon la formule = (Masse des composés volatils /Masse initiale de l'extrait) x 100.

En appliquant cette formule on a déterminé que l'extrait selon l'invention comprend de 25 à 50% en poids de composés volatils.

*Analyse et résultats*

Le tableau 1 présente les principaux composés volatils obtenus par GC/FID et le pourcentage en poids de chacun.

[Tableau 1]

| Tr | Composés | % Fid |
|---|---|---|
| 4.26 | Acide Acétique | 0.235 |
| 5.40 | Methallyl Cyanide | 0.082 |
| 8.67 | Cyano-Pentène* | 0.065 |
| 8.97 | 2-Furanméthanol | 0.026 |
| 13.20 | 2,4-Dihydroxy-2,5-dimethyl-3(2H)-furan-3-one | 0.034 |
| 18.79 | 4H-Pyran-4-one, 2,3-dihydro-3,5-dihydroxy-6-methyl- | 0.140 |
| 22.00 | 2(E)-Décénal | 0.020 |
| 23.50 | 2-Méthoxy-4-Vinylphénol | 0.089 |
| 30.33 | 4-Ethényl-2,6-Diméthyl-Phénol | 4.327 |
| 37.70 | Palmitate de méthyle | 0.213 |
| 38.55 | Acide Palmitique | 6.766 |
| 39.09 | Palmitate d'Ethyle | 0.515 |
| 41.13 | Linoléate de méthyle | 1.214 |
| 41.30 | Oléate de méthyle | 2.611 |
| 42.62 | Oléate d'Ethyle | 7.301 |
| 44.04 | Acide Oléique | 70.061 |
| 44.11 | Acide Stéarique | 0.350 |
| 47.46 | Céramide/ amide gras 1 | 0.371 |
| 47.56 | Céramide/ amide gras 2 | 0.723 |
| 48.45 | Céramide/ amide gras 3 | 1.105 |
| | Total | 96.248 |

Tr correspond au temps de rétention du composé volatil.
D'après le tableau 1, la phase des composés volatils comprend 2,2 % poids de céramides et/ou amides gras.

B. Analyse de la teneur en protéines de l'extrait par la méthode de Bradford

Pour cette analyse, l'extrait désolvaté obtenu à l'exemple 1 a été dilué dans de l'éthanol à 96°selon un rapport 1/1.
Les quantités de protéines totales présentes ont été déterminées par dosage colorimétrique selon la méthode de Bradford.
Pour ce dosage colorimétrique, une courbe étalon de la densité optique, mesurée à 595 nm en fonction de la quantité de protéines a été réalisée. La protéine de référence utilisée dans cette expérience est la sérum-albumine bovine (BSA).
Puis la détermination de la quantité totale en protéines dans chaque échantillon de l'extrait à tester a été obtenue en préparant des échantillons à tester selon le même protocole que celui utilisé pour la gamme étalon puis en reportant les valeurs de DO (densité optique) obtenues pour les échantillons à tester sur la courbe étalon.

*Préparation de la gamme étalon*

Les réactifs utilisés pour préparer la gamme étalon étaient les suivants :

**BSA** : Serum albumine bovine commercialisé par Sigma Aldrich ® ;

**Eau** : WATER PLUS commercialisée par Carlo Erba ® ;

**Réactif de Bradford** commercialisé par Sigma Aldrich ® ;

**DMSO** commercialisé par Sigma Aldrich ®.

Les solutions suivantes ont été préparées :

**BSA à 2 mg/mL** : solubilisation de 2 mg de BSA dans 1mL d'eau WATER PLUS ;

**BSA à 0,2 mg/ml** : dilution de 100 $\mu$L de la solution de BSA à 2 mg/mL dans 1mL (volume final) d'eau WATER PLUS.

La gamme étalon a été réalisée en trois exemplaires (triplicate), les compositions formant la gamme étalon sont présentées au tableau 2.

[Tableau 2]

| N° de tube | 1 | | 2 | 3 | 4 | 5 | 6 | 7 |
|---|---|---|---|---|---|---|---|---|
| Quantité de BSA ($\mu$g) | 0 | | 1 | 2 | 4 | 6 | 8 | 10 |
| H20 (mL) | | qsp1mL | | | | | | |

*Protocole*

Les quantités de BSA et d'eau indiquées dans le tableau ci-dessus ont été introduites dans les tubes 1 à 7, puis 1mL de réactif de Bradford a été ajouté dans chaque tube enfin les tubes ont été mélangés par retournement. Après un temps de pause de 30 minutes à température ambiante, les mesures de densité optique à 595 nm ont été réalisées au moyen d'un spectrophotomètre « Genesys 150 » de la société ThermoFisher Scientific.

Les mesures de densité optique à 595 nm ont permis de définir une courbe d'équation : *y = 0,0315x avec R$^2$ = 0,9817.*

Les échantillons à tester ont été réalisés à partir de l'extrait à 50% d'éthanol à 96°, l'extrait comprend donc 50% v/v d'extrait de tourteau de colza de l'exemple 1.

Ainsi, les échantillons à tester ont été préparés selon le protocole suivant : les échantillons sont dilués dans le DMSO selon des dilutions d'un facteur allant de 10 à 200. Puis de l'eau (qsp 1 mL) et enfin 1 mL de réactif de Bradford et ont a été ajoutés. Les tubes ont été mélangés par retournement, puis après une pause de 30 minutes à température ambiante, les mesures de densité optique à 595 nm ont été réalisées au moyen d'un spectrophotomètre « Genesys 150 » de la société ThermoFisher Scientific.

Dans le tableau 3 sont présentés les résultats obtenus pour une dilution des échantillons égale à 100. A partir de la courbe étalon, la concentration en protéines de chaque échantillon a été déterminée.

[Tableau 3]

| Volume testé | DO 595 nm | Quantité de protéines ($\mu$g) | Concentration en protéines (dilué) (mg/mL) | Concentration en protéines (mg/mL) |
|---|---|---|---|---|
| 50 | 0,156 | 4,95 | 0,099 | 9,90 |
| 50 | 0,147 | 4,67 | 0,093 | 9,33 |
| 50 | 0,155 | 4,92 | 0,098 | 9,84 |
| 75 | 0,253 | 8,03 | 0,107 | 10,71 |
| 75 | 0,251 | 7,97 | 0,106 | 10,62 |
| 75 | 0,244 | 7,75 | 0,103 | 10,33 |
| 25 | 0,076 | 2,41 | 0,097 | 9,65 |
| 25 | 0,078 | 2,48 | 0,099 | 9,90 |

A partir du tableau 3, on a déduit que l'extrait à 50% d'éthanol présente une teneur en protéines de 10,04 $\pm$ 0,48 mg/mL, l'extrait selon l'invention présente donc une teneur en protéines de 20,08 $\pm$ 0,96mg/mL, soit 2% poids.

C. Analyse de la teneur en tocophérols de l'extrait par détection à ionisation de flamme

L'extrait obtenu à l'exemple 1 est extrait à l'hexane (ratio 1 :1). L'extrait obtenu est analysé par chromatographique gazeuse par détection à ionisation de flamme (GC/FID).

Selon cette méthode, l'échantillon est préparé selon une méthode de micro-extraction en phase solide dite SPME qui utilise comme support solide de piégeage la fibre CAR/PDMS (Carboxen®/Polydimethylsiloxane fibers). Le piègeage a lieu pendant 30 minutes à 90°C.

Il a été établi que l'extrait selon l'invention comprend également de 25 à 50% en poids de composés volatils miscibles dans l'hexane.

*Analyse et résultats*

Le tableau 4 présente les principaux composés volatils, miscibles dans l'hexane obtenus par GC/FID et le pourcentage en poids de chacun.

[Tableau 4]

| Tr | Composés | % Fid |
|---|---|---|
| 22.00 | 2(E)-Décénal | 0.166 |
| 22.41 | D-Glucitol, 1,4:3,6-dianhydro-2,5-di-O-methyl- | 0.014 |
| 22.97 | (2E,4Z)-Décadiènal | 0.025 |
| 23.64 | (2E,4E)-Décadiènal | 0.046 |
| 30.06 | 4-Ethényl-2,6-Diméthyl-Phénol | 1.238 |
| 37.70 | Palmitate de méthyle | 0.092 |
| 39.10 | Acide Palmitique | 7.166 |
| 41.00 | Linoléate de méthyle | 0.400 |
| 41.15 | Oléate de méthyle | 0.975 |
| 42.37 | Oléate d'Ethyle | 0.676 |
| 43.40 | Acide Oléique | 83.342 |
| 43.48 | Acide Stéarique | 1.355 |
| 47.41 | Oléate de Diméthylaminoethyle | 0.555 |
| 50.10 | gamma-Tocophérol | 0.649 |
| 52.46 | Vitamine E | 0.560 |
|  | Total | 97.259 |

Cette analyse a permis de déterminer les teneurs en tocophérols de la phase des composés volatils : la teneur en gamma tocophérols est de 0,65 % poids et en alpha tocophérols (Vitamine E) est de 0,56 % en poids par rapport au poids des composés volatils miscibles dans l'hexane.

D. Analyse de la teneur des polyphénols totaux de l'extrait par la méthode utilisant le réactif de Folin Ciocalteu

Cette analyse a été effectuée à partir de l'extrait de l'exemple 1 dilué dans de l'éthanol 96° selon un rapport 1/1.

Le dosage des polyphénols totaux a été effectué selon la méthode décrite en 1965 par Singleton et Rossi en utilisant le réactif de Folin-Ciocalteu. Le réactif de Folin Ciocalteau est un acide de couleur jaune constitué par un mélange d'acide phosphotungstique ($H_3PW_{12}O_{40}$) et d'acide phosphomolybdique ($H_3PMo_{12}O_{40}$). Il est réduit, lors de l'oxydation des phénols, en un mélange d'oxydes bleus de tungstène et de molybdène (Ribereau, 1968). La coloration produite, dont l'absorption maximale à 720nm, est proportionnelle à la quantité de polyphénols présents dans les extraits végétaux.

Pour ce dosage colorimétrique, une courbe étalon de la densité optique, mesurée à 720 nm, en fonction de la quantité de polyphénols a été réalisée.

Le composé de référence utilisé dans cette expérience est l'acide gallique.

Préparation de la gamme étalon

Les ingrédients pour préparer la gamme étalon étaient les suivants :

Solution de Na2CO3 (bicarbonate de soude) : Peser 15g de bicarbonate de soude (commercialisé par Solvay ®) dans une fiole jaugée 200ml et compléter avec de l'eau distillée.
Réactif de Folin-Ciocalteu commercialisé par Sigma ® : Diluer le réactif à 10% dans de l'eau
Acide gallique : préparation de la solution mère à 0,1g/L : Peser 0,01 g d'acide gallique poudre dans une fiole jaugée de 100ml et ajuster avec l'eau distillée.

La gamme étalon a été réalisée en trois exemplaires avec des solution d'acide gallique de concentration allant de 1 à 6 mg/L.
Les mesures de densité optique à 720 nm ont permis de définir une courbe d'équation : $y = 0,1247x - 0,173$ avec $R^2 = 0,9759$.
Puis la détermination de la quantité totale en polyphénols dans chaque échantillon d'hydrolysat à tester a été obtenue en préparant des échantillons à tester selon le même protocole que celui utilisé pour la gamme étalon puis en reportant les valeurs de DO (densité optique) obtenues pour les échantillons à tester sur la courbe étalon.
On a déduit que l'extrait à 50% d'éthanol présente une teneur en polyphénols de 67.76 mg/L soit une valeur de 135 mg/L (135 ppm) dans l'extrait selon l'invention.

## Exemple 2 - Cytotoxicité

Ces analyses ont été effectuées à partir d'une solution de l'extrait de l'exemple 1 dilué dans de l'éthanol 96° selon un rapport 1/1 : la solution 50/50. Pour effectuer les mesures, des compositions de l'extrait selon l'invention à différentes concentrations, ont été préparées en diluant la solution 50/50 directement dans le milieu d'incubation à la concentration visée.
Les cytotoxicités de ces compositions de l'extrait de tourteau de colza de l'exemple 1 à des concentrations différentes ont été évaluées sur des mélanocytes normaux d'épiderme humain cultivés en culture monocouche.
Avant de procéder aux mesures, les compositions d'extrait de tourteau de colza ont été conservées à température ambiante, à l'abri de la lumière.
Les mélanocytes normaux d'épiderme humain ont été cultivés en culture monocouche jusqu'à confluence. Puis les mélanocytes ont été incubés pendant 72 heures soit en l'absence de l'extrait à tester (contrôle) soit en présence des différentes compositions de l'extrait à tester.
La viabilité des mélanocytes a été déterminée par une méthode spectro-colométrique basée sur des mesures d'activité phosphatase alcaline.
Le principe de cette mesure repose sur la transformation du substrat p-nitrophényl phosphate en p-nitrophénol par la phosphatase alcaline intracellulaire des cellules viables, selon la méthode décrite dans « An acid phosphatase assay for quantifying the growth of adherent and nonadherent cells. » Yang T., Sinai P., Kain S. Anal. Biochem., 24, 103-108 (1996).
Dans le tableau 5, les résultats obtenus sont exprimés en pourcentage de cellules viables par rapport au contrôle, la moyenne ainsi que la déviation standard (DS) sont également présentées.

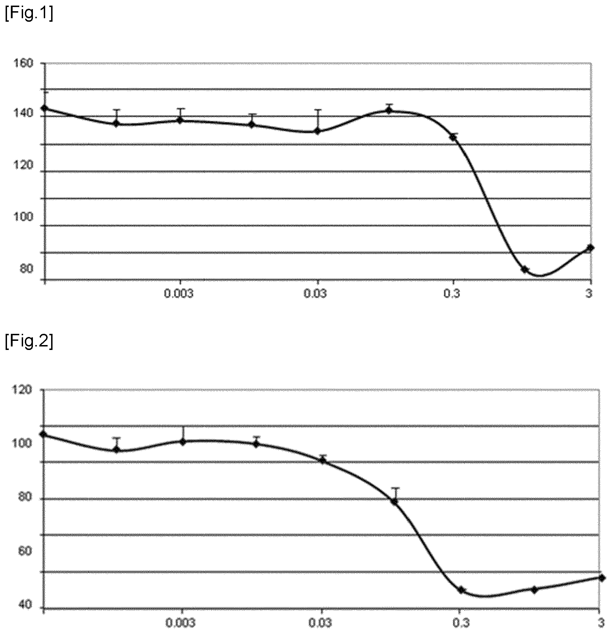

[Fig.1] représente le pourcentage de cellules (mélanocytes) viables par rapport au contrôle en fonction de la concentration de la composition d'extrait de tourteau de colza (vol/vol).

[Tableau 5]

|  | Contrôle | % (v/v) d'extrait de l'exemple 1 | | | |
|---|---|---|---|---|---|
|  |  | 0,0003% | 0,001 % | 0,003% | 0,01% |
| % de cellules viables | 99.2 | 112.2 | 102.4 | 106.3 | 104.5 |
|  | 108.6 | 132.3 | 119.8 | 122.0 | 119.5 |
|  | 92.2 | 133.6 | 122.2 | 123.1 | 117.7 |
| **Moyenne** | **100.0** | **126.0** | **114.8** | **117.1** | **113.9** |

(suite)

| D.S. | 8.2 | 12.0 | 10.8 | 9.4 | 8.2 |
|------|-----|------|------|-----|-----|
| % (v/v) d'extrait de l'exemple 1 | | | | | |
| % de cellules viables | 0,03% | 0,1% | 0,3% | 1 | 3 |
| | 91.9 | 128.9 | 104.9 | 6.7 | 21.2 |
| | 121.8 | 123.7 | 107.3 | 7.1 | 24.7 |
| | 115.1 | 120.3 | 101.5 | 7.6 | 24.1 |
| **Moyenne** | **109.6** | **124.3** | **104.5** | **7.2** | **23.3** |
| D.S. | 15.7 | 4.3 | 2.9 | 0.5 | 1.8 |

Il ressort de cette étude que l'extrait de tourteau de colza présente un effet cytotoxique sur les mélanocytes normaux d'épiderme humain détectable à partir de la concentration à 1% v/v.

*Sur kératinocytes*

Les kératinocytes normaux d'épiderme humain d'un donneur de 66 ans ont été cultivés en culture monocouche jusqu'à confluence. Puis les kératinocytes ont été incubés pendant 48 heures soit en l'absence de l'extrait à tester (contrôle) soit en présence des différentes compositions de l'extrait à tester.

La viabilité des kératinocytes a été déterminée par la méthode spectro-colométrique basée sur des mesures d'activité phosphatase alcaline de Yang *et al* décrite pour les mélanocytes ci-dessus.

Dans le tableau 6, les résultats obtenus sont exprimés en pourcentage de cellules viables par rapport au contrôle, la moyenne ainsi que la déviation standard (DS) sont également présentées.

[Fig.2] représente le pourcentage de cellules viables par rapport au contrôle en fonction de la concentration de la composition d'extrait de tourteau de colza (vol/vol).

[Tableau 6]

| | Contrôle | % (v/v) d'extrait de l'exemple 1 | | | |
|---|---|---|---|---|---|
| | | 0,0003% | 0,001 % | 0,003% | 0,01% |
| % de cellules viables | 100.6 | 89.4 | 82.1 | 87.4 | 86.9 |
| | 96.7 | 96.1 | 83.4 | 86.3 | 89.6 |
| | 102.7 | 99.7 | 94.1 | 101.4 | 94.5 |
| **Moyenne** | **100.0** | **95.1** | **86.5** | **91.7** | **90.3** |
| D.S. | 3.0 | 5.2 | 6.5 | 8.4 | 3.9 |
| | % (v/v) d'extrait de l'exemple 1 | | | | |
| % de cellules viables | 0,03% | 0,1% | 0,3% | 1 | 3 |
| | 79.1 | 58.1 | 9.6 | 10.3 | 12.8 |
| | 79.8 | 66.8 | 10.3 | 10.8 | 19.6 |
| | 84.5 | 51.2 | 10.9 | 10.8 | 17.9 |
| **Moyenne** | **81.1** | **58.7** | **10.3** | **10.6** | **16.8** |
| D.S. | 3.0 | 7.8 | 0.6 | 0.3 | 3.5 |

Il ressort de cette étude que l'extrait de tourteau de présente un effet cytotoxique sur les kératinocytes normaux d'épiderme humain détectable à partir de la concentration à 0,1% v/v. En outre des modifications de la morphologie des cellules, reflétant un stress, ont été observées à partir de la concentration à 0,1% v/v.

Cette différence avec les résultats sur mélanocytes est conforme aux attentes car les kératinocytes appartiennent aux couches supérieures de l'épiderme, et sont donc naturellement plus sensibles aux céramides qui constituent le ciment intercellulaire des couches les plus supérieures, les mélanocytes appartenant à la couche basale de l'épiderme ont moins de sensibilité vis-à-vis des céramides.

Il ressort de ces études de cytotoxicité *in vitro* que des compositions cosmétiques comprenant jusqu'à 30 % en poids d'extrait selon l'invention peuvent être appliquées sur la peau.

**Exemple 3 Mesures des activités cosmétiques**

**1. Activité propigmentante**

**Mesure de l'augmentation de production de la mélanine**

Les mesures de l'activité propigmentante (augmentation de la production de mélanine) de plusieurs compositions de l'extrait de tourteau de colza présentant des concentrations différentes ont été effectuée par dosage des mélanocytes humains normaux *in vitro*.

Les compositions de concentrations 0,03% (v/v), 0,1% (v/v), et 0,3% (v/v) ont été préparées par dilution de la solution 50/50 dans le milieu d'incubation.

Les cultures des mélanocytes normaux d'épiderme humain ont été cultivés en culture monocouche jusqu'à confluence. Puis les mélanocytes ont été incubés pendant 72 heures soit en l'absence de l'extrait à tester (contrôle) soit en présence des différentes compositions de l'extrait à tester. Le produit de référence pour l'inhibition de la synthèse de mélanine était une solution d'acide kojique à 250 $\mu$M.

Les quantités de mélanine produite par les mélanocytes ont été déterminées par une méthode spectrophotométrique c'est-à-dire une mesure de densité optique à 405 nm effectuée au moyen d'un spectrophotomètre « Genesys 150 » de la société ThermoFisher Scientific.

Le tableau 7 présente la quantité de mélanine ($\mu$g) produite par mg de protéines totales dans le lysat cellulaire monocouche.

Le niveau de signification entre le "contrôle" et la « référence » a été évalué à l'aide d'un test t de Student (* : p<0,05).

[Tableau 7]

| | Contrôle | Concentration de l'extrait de l'exemple 1 (% v/v) | | | Référence acide kojique à 250 $\mu$M. |
|---|---|---|---|---|---|
| | | 0,03% | 0,1% | 0,3% | |
| $\mu$g mélanine/mg de protéines | 64.7 | 77.0 | 84.4 | 125.2 | 45.1 |
| | 64.5 | 52.6 | 81.1 | 124.2 | 30.3 |
| | 75.8 | 56.8 | 124.0 | 136.6 | 32.0 |
| **Moyenne** | **68.3** | **62.1** | **96.5** | **128.7**** | **35.8**** |
| D.S. | 6.5 | 13.1 | 23.9 | 6.9 | 8.1 |
| **% du contrôle** | **100** | **90.9** | **141.2** | **188.3** | **52.4** |
| **: signifie différent de manière significtive du "Control" (p<0.01). | | | | | |

On observe en particulier que l'extrait de tourteau de colza à la concentration de 0,3% augmente de manière significative la production de mélanine de 88,3%. L'échantillon à 0,1% confirme la tendance propigmentante de l'extrait selon l'invention. Le produit de référence inhibe de manière significative la production de mélanine de 47,6% ce résultat était attendu et valide ce test.

Les compositions selon la présente invention présentent donc un effet propimentant, stimulant la synthèse de la mélanine, en application sur la peau ces compositions permettent de conférer à leurs utilisateurs un effet « bonne mine ».

**2.Activité lipolytique**

**A/ Mesure de l'augmentation de l'activité lipolytique**

Les mesures de l'activité lipolytique de plusieurs compositions d'extrait de tourteau de colza à des concentrations différentes ont été effectuées sur des adipocytes humains normaux de tissu cutané adipeux prélevé sur un adulte de 40 ans. Une suspension des adipocytes humains a été incubée pendant 2 H en présence d'une part des compositions d'extrait à tester et d'autre part du produit de référence (contrôle).

Les compositions de concentrations 0,003% (v/v), 0,01% (v/v), et 0,03% (v/v) d'extrait de tourteau de colza ont été préparées par dilution de la solution 50/50 dans le milieu d'incubation.

Le produit de référence utilisé pour déterminer l'activité lypolytique était la caféine aux concentrations suivantes $10^{-4}$ mol/L et $10^{-3}$ mol/L.

*Mesures de la concentration NEFA (acides gras non estérifiés)*

À la fin de la période d'incubation, la quantité de NEFA libérés dans le milieu d'incubation a été déterminée à l'aide d'un kit de mesure spectro-colométrique spécifique et sensible.

Le tableau 8 présente les résultats en $\mu$M de NEFA et en pg/ml mesuré dans le milieu d'incubation des adipocytes, sont également présentées les moyenne et déviation standard SD.

Le niveau de signification entre le "contrôle" et le "produit de référence" a été évalué à l'aide d'un test t de Student (* : $p < 0,05$).

Le niveau de signification entre le le "contrôle" et chaque "composé testé" a été évalué indépendamment pour chaque composition par une analyse de variance à un facteur (ANOVA à sens unique) suivie d'un test de Holm-Sidak (* : $p < 0,05$).

[Tableau 8]

| | contrôle | Concentration de l'extrait de l'exemple 1 (%v/v) | | | Caféine | |
|---|---|---|---|---|---|---|
| | | 0.003 | 0.01 | 0.03 | $10^{-4}$ mol/L | $10^{-3}$ mol/L |
| NEFA ($\mu$M) | 31.5 | 36.9 | 31.7 | 40.8 | 37.1 | 41.5 |
| | 34.9 | 37.5 | 37.1 | 40.6 | 38.8 | 50.3 |
| | 35.4 | 36.7 | 35.3 | 42.8 | 37.2 | 51.9 |
| **Moyenne** | **33.9** | **37.0** | **34.7** | **41.4\*\*** | **37.7\*** | **47.9\*** |
| D.S. | 2.1 | 0.4 | 2.8 | 1.2 | 0.9 | 5.6 |
| % du contrôle | 100 | 109.1 | 102.3 | 122.0 | 111.1 | 141.1 |
| *: signifie différent de manière significative du "Control" ($p < 0.05$) et **: signifie différent de manière significative du "Control" ($p < 0.01$). | | | | | | |

On observe que l'extrait de tourteau de colza selon l'invention à la concentration de 0,03% augmente de manière significative la quantité d'acides gras non estérifiés libérés par les adipocytes humains, la lipolyse des adipocytes est augmentée de 22% du contrôle ($p < 0.01$).

Les autres échantillons présentent également une augmentation de la quantité de NEFA et confirment la tendance de la stimulation de la lypolyse de l'extrait selon l'invention sur les adipocytes humains normaux.

Le produit de référence, la caféine, testée à $10^{-4}$ mol/L et $10^{-3}$ mol/L augmente de manière significative la lipolyse des adipocytes respectivement de 11.1% ($p < 0.05$) et 41.1% ($p < 0.05$), ce résultat était attendu et valide ce test d'augmentation de la lipolyse des adipocytes.

En application sur la peau, l'extrait de tourteau de colza selon l'invention présente des propriétés amincissantes.

En application sur la peau, la composition présente un effet amincissant notamment au niveau du contour du visage. Le visage paraît à la fois mieux dessiné.

Cet effet minceur a été confirmé par la tendance de l'extrait selon l'invention à stimuler la production de ZAG sur des kératinocytes humains normaux.

**3.Activité relaxante et anti-stress pour la peau**

L'extrait selon l'invention a été testé sur l'enzyme clé de la cascade arachidonique Phospholipase A2 (PLA2, enzyme notamment présente dans la peau.

Les compositions de concentrations 0,001% (v/v), 0,01 % (v/v), et 0,1% (v/v) ont été préparées par dilution de la solution 50/50 dans le milieu d'incubation.

Les résultats en termes de moyenne, moyenne moins le blanc, % d'activité enzymatique et % d'inhibition sont présentés dans le tableau 9.

[Tableau 9]

| Echantillon | Blanc | T+ | T- | 0,1% | 0,01% | 0,001 % |
|---|---|---|---|---|---|---|
| Moyenne | 0,176 | 2,338 | 0,387 | 2,257 | 2,738 | 1,933 |
| Moyenne - blanc | 0,000 | 2,163 | 0,211 | 1,355 | 2,507 | 1,726 |
| % activité enzymatique | 0,00 | 100,00 | 9,76 | 62,64 | 115,91 | 97,64 |
| % inhibition | 100% | 0% | 90% | 37% | Non interpretable | 2% |

L'extrait de tourteau de Colza inhibe de manière significative la PLA2 dès la dose de 0,1% avec un score à 37% d'inhibition.

En application sur la peau, l'extrait selon l'invention produit un effet de peau reposée, apaisée.

**Exemple 4- Compositions cosmétiques et alimentaires**

Les compositions selon l'invention suivantes ont été réalisées.

4.1 Crème de corps apaisante/relaxante

[Tableau 10]

| Composant | g |
|---|---|
| Extrait de tourteau de colza selon l'exemple 1 | 1,0 |
| Urée (UREE PERLES 46% commercialisé par Brenntag ®) | 1,0 |
| Acide lactique (80% M.A.) commercialisé par Prochimia | 0,2 |
| Acide salicylique commercialisé par Novacyl | 0,3 |
| Benzoate de sodium commercialisé par Prochimia | 0,5 |
| Gomme de xanthane (FFPC commercialisé par Jungbunzlauer ®) | 0,2 |
| Glycérine végétale (Palmera G995E commercialisé par Interchimie ®) | 3,5 |
| Bentonite/ gomme de xanthane/stéaroyle de sodium glutamate / acide citrique (Frametime CXG commercialisé par Ephyla) | 3,2 |
| Alcool cétostéarylique 50/50 commercialisé par Interchimie | 1,0 |
| Beurre de karité commercialisé par Ephyla | 1,5 |
| Huile de colza commercialisée par centre Ouest Céréales | 16,5 |
| Parfum commercialisé par Robertet | 0,5 |
| Eau | 70,7 |

En application régulière, cette composition permet un effet amincissant, relaxant de la peau, elle permet aussi un léger effet bronzant.

4.2 Crème solaire propigmentante

[Tableau 11]

| Composant | g |
|---|---|
| Extrait de tourteau de colza selon l'exemple 1 | 2,00 |
| Bentonite/ gomme de xanthane/stéaroyle de sodium glutamate / acide citrique (Frametime CXG commercialisé par Ephyla) | 3,00 |

(suite)

| Composant | g |
|---|---|
| Bentonite et extrait de Ulva lacttuca (Ulvaprotect commercialisé par Ephyla) | 1,00 |
| Sel fin raffiné séché commercialisé par Terre de Sel | 1,00 |
| Vitamine D3 Like commercialisée par Ephyla | 1,00 |
| Glycérine végétale (Palmera G995E commercialisé par Interchimie ®) | 4,00 |
| Propanediol commercialisé par DuPont Tate & Lyle | 4,00 |
| Huile de dattier du désert commercialisée par Ephyla | 3,00 |
| Huile de graine d'Helianthus annuus et gomme de Canarium luzonicum (HTRE commercialisé par Ephyla) | 2,00 |
| Canolate de méthyle (Ester MC R commercialisé par Ephyla) | 17,00 |
| Polyglyceryl-8- oléate (NS-EX-81 commercialisé par Next Step Lab) | 1,00 |
| Alcool cétostéarylique 50/50 commercialisé par Interchimie | 2,00 |
| Oxyde de zinc/acide polyhydroxystéarique (Super Zinc Sheer Natural commercialisé par Next Step Lab) | 20,00 |
| Alcool benzylique commercialisé par MLW | 2,00 |
| Eau | 38,00 |

Cette crème solaire auto bronzante confère également un effet amincissant.

4.3 Shampooing douche relaxant

[Tableau 12]

| Composant | g |
|---|---|
| Extrait de tourteau de colza selon l'exemple 1 | 1,000 |
| Bentonite/ gomme de xanthane/stéaroyle de sodium glutamate / acide citrique (Frametime CXG commercialisé par Ephyla) | 4,725 |
| Coco-sulfate de sodium (SULFETAL C90C commercialisé par Masso) | 5,100 |
| Isethionate de cocoyl sodium (ELFAN AT 84 commercialisé par Masso) | 4,050 |
| Acide salicylique commercialisé par Novacyl | 0,300 |
| Benzoate de sodium commercilisé par Prochimia | 0,495 |
| Gomme de xanthane (FFPC commercialisé par Jungbunzlauer) | 0,105 |
| Pigments (COVAPEARL ANTIQUE SILVER 239 commercialisé par Sensient) | 0,225 |
| Parfum commercialisé par Robertet | 0, 500 |
| Eau | 83,500 |

En application régulière, cette composition pour la douche permet un effet amincissant, relaxant, anti-stress de la peau.

4.4 Huile sèche apaisante

[Tableau 13]

| Composant | g |
|---|---|
| Extrait de tourteau de colza selon l'exemple 1 | 2,00 |
| Methyl Ester de Colza (EPHYSTER MCR commercialisé par Ephyla) | 43,00 |
| Huile de graine d'Helianthus annuus et Résine de Protium heptaphyllum (HTR1 commercialisé par Ephyla) | 2,00 |
| Huile de colza commercialisée par Centre Ouest Céréales | 51,00 |
| Parfum commercialisé par Robertet | 2,00 |

Cette huile sèche confère un effet auto bronzant et amincissant.

4.5 Composition alimentaire : huile de bouche minceur

[Tableau 14]

| Composant | g |
|---|---|
| Extrait de tourteau de colza selon l'exemple 1 | 3,00 |
| Acide linoléique conjugué commercialisé par Ephyla | 3,00 |
| Huile de colza commercialisée par Centre Ouest Céréales | 93,90 |
| Vitamine E (NUTRABIOL E) commercialisée par BTSA | 0,1 |

4.6 Complément alimentaire minceur poudre pour gélules 750 à 1000mg/gélule

[Tableau 15]

| Composant | g |
|---|---|
| Extrait de tourteau de colza selon l'exemple 1 | 5,00 |
| Acide linoléique conjugué commercialisé par Ephyla | 95,00 |

**Revendications**

1. Extrait de tourteau de colza **caractérisé en ce qu'**il est susceptible d'être obtenu par un procédé de préparation comprenant au moins les étapes suivantes, dans cet ordre :

- fournir des graines de colza,
- presser les graines de colza et récupérer le tourteau,
- broyer le tourteau pour obtenir une taille moyenne des particules inférieure ou égale 5 mm,
- procéder à l'extraction du tourteau de colza au moyen d'éthanol à 96°,
- filtrer,
- récupérer le filtrat,
- évaporer le solvant,
- récupérer l'extrait obtenu,

ledit extrait de tourteau de colza comprenant de 0,17 % à 0,33 % en poids de gamma-tocophérols par rapport au poids total de l'extrait et de 0,14 % à 0,28 % en poids d'alpha-tocophérols par rapport au poids total de l'extrait .

2. Extrait de tourteau de colza selon la revendication 1 **caractérisé en ce qu'**il comprend de 1 à 5% de préférence 2 % en poids de protéines par rapport au poids total de l'extrait.

**3.** Extrait de tourteau de colza selon l'une quelconque des revendications précédentes **caractérisé en ce qu'**il présente une teneur en polyphénols allant de 100 à 150 ppm, et de préférence 135 ppm.

**4.** Extrait de tourteau de colza selon l'une quelconque des revendications précédentes **caractérisé en ce qu'**il comprend de 50 à 75% en poids d'huile de colza et de 25 à 50% en poids de composés volatils, par rapport au poids total de l'extrait.

**5.** Extrait de tourteau de colza selon l'une quelconque des revendications précédentes **caractérisé en ce qu'**il comprend de 0,5 à 1,25 %, de préférence 0,9% en poids de composés choisis parmi les céramides et les amides gras en $C_8$-$C_{24}$ par rapport au poids total de l'extrait.

**6.** Extrait de tourteau de colza selon l'une quelconque des revendications précédentes **caractérisé en ce qu'**il comprend 0,25 % en poids de gamma-tocophérols par rapport au poids total de l'extrait et 0,20 % en poids d'alpha-tocophérols par rapport au poids total de l'extrait.

**7.** Procédé de préparation de l'extrait selon l'une quelconque des revendications précédentes comprenant au moins les étapes suivantes, dans cet ordre :

- fournir des graines de colza,
- presser les graines de colza et récupérer le tourteau,
- broyer le tourteau pour obtenir une taille moyenne des particules inférieure ou égale 5 mm,
- procéder à l'extraction du tourteau de colza au moyen d'éthanol à 96°,
- filtrer,
- récupérer le filtrat,
- évaporer le solvant,
- récupérer l'extrait obtenu.

**8.** Procédé selon la revendication précédente dans lequel, lors de l'extraction, le ratio masse de tourteau de colza / masse d'éthanol est compris entre 1/1 et 1/4 et de manière préférée d'environ 1/2.

**9.** Procédé selon la revendication 7 ou 8 **caractérisée en ce que** l'extraction est effectuée pendant une durée allant de 1,5 à 4 heures, à une température allant de 12 à 45°C.

**10.** Composition, non thérapeutique, cosmétique ou alimentaire, **caractérisée en ce qu'**elle comprend au moins un extrait selon l'une quelconque des revendications 1 à 6 ou préparé suivant le procédé selon l'une quelconque des revendications 7 à 9, de préférence en une teneur allant de 0,001 à 30 % en poids et de préférence de 0,1 à 20 % en poids par rapport au poids total de la composition.

**11.** Composition cosmétique, non thérapeutique selon la revendication précédente comprenant, dans un milieu cosmétiquement acceptable, au moins un agent cosmétique choisi parmi les agents humectants, les épaississants, les agents de texture, les émulsifiants, les agents dispersants, les agents moussants, les émolients, les conservateurs, les colorants, les extraits de plantes, les fibres végétales, les minéraux, les agents correcteurs de pH, les principes actifs et les parfums.

**12.** Composition cosmétique selon l'une quelconque des revendications 10 ou 11 **caractérisée en ce qu'**elle est destinée à être appliquée sur la peau.

**13.** Utilisation cosmétique, non thérapeutique, d'au moins un extrait selon l'une quelconque des revendications 1 à 6 ou préparé suivant le procédé selon l'une quelconque des revendications 7 à 9, ou d'au moins une composition cosmétique selon l'une quelconque des revendications 10 à 12 en tant qu'actif propigmentant, autobronzant.

**14.** Utilisation cosmétique, non thérapeutique, d'au moins un extrait selon l'une quelconque des revendications 1 à 6 ou préparé suivant le procédé selon l'une quelconque des revendications 7 à 9, ou d'au moins une composition cosmétique selon l'une quelconque des revendications 10 à 12 en tant qu'actif amincissant notamment pour affermir la peau du contour du visage.

**15.** Procédé de traitement cosmétique, non thérapeutique de la peau, comprenant au moins une étape d'application sur la peau, d'au moins un extrait selon l'une quelconque des revendications 1 à 6 ou préparé suivant le procédé selon l'une quelconque des revendications 7 à 9, ou d'au moins une composition cosmétique selon l'une quelconque des revendications 10 à 12.

**Patentansprüche**

1. Rapskuchenextrakt, **dadurch gekennzeichnet, dass** er durch ein Zubereitungsverfahren gewinnbar ist, das mindestens die folgenden Schritte in dieser Reihenfolge umfasst:

   - Bereitstellen der Rapssamen,
   - Pressen der Rapssamen und Zurückgewinnen des Kuchens,
   - Zerkleinern des Kuchens, um eine durchschnittliche Partikelgröße von kleiner oder gleich 5 mm zu erhalten,
   - Extrahieren des Rapskuchens mit Ethanol bei 96 °,
   - Filtern,
   - Zurückgewinnen des Filtrats,
   - Verdampfen des Lösungsmittels,
   - Zurückgewinnen des gewonnenen Extrats,

   wobei der Rapskuchenextrakt 0,17 bis 0,33 Gew.-% Gamma-Tocopherole bezogen auf das Gesamtgewicht des Extrakts und 0,14 bis 0,28 Gew.-% Alpha-Tocopherole bezogen auf das Gesamtgewicht des Extrakts umfasst.

2. Rapskuchenextrakt nach Anspruch 1, **dadurch gekennzeichnet, dass** er von 1 bis 5, vorzugsweise 2 Gew.-% Proteine bezogen auf das Gesamtgewicht des Extrakts umfasst.

3. Rapskuchenextrakt nach einem der vorhergehenden Ansprüche, **dadurch gekennzeichnet, dass** er einen Polyphenolgehalt von 100 bis 150 ppm und vorzugsweise von 135 ppm aufweist.

4. Rapskuchenextrakt nach einem der vorhergehenden Ansprüche, **dadurch gekennzeichnet, dass** er 50 bis 75 Gew.-% Rapsöl und 25 bis 50 Gew.-% flüchtige Verbindungen bezogen auf das Gesamtgewicht des Extrakts umfasst.

5. Rapskuchenextrakt nach einem der vorhergehenden Ansprüche, **dadurch gekennzeichnet, dass** er 0,5 bis 1,25 Gew.-%, vorzugsweise 0,9 Gew.-% Verbindungen umfasst, die aus Ceramiden und $C_8$-$C_{24}$-Fettamiden bezogen auf das Gesamtgewicht des Extrakts ausgewählt sind.

6. Rapskuchenextrakt nach einem der vorhergehenden Ansprüche, **dadurch gekennzeichnet, dass** er 0,25 Gew.-% Gamma-Tocopherole bezogen auf das Gesamtgewicht des Extrakts und 0,20 Gew.-% Alpha-Tocopherole bezogen auf das Gesamtgewicht des Extrakts umfasst.

7. Verfahren zur Zubereitung des Extrakts nach einem der vorhergehenden Ansprüche, das mindestens die folgenden Schritte in dieser Reihenfolge umfasst:

   - Bereitstellen der Rapssamen,
   - Pressen der Rapssamen und Zurückgewinnen des Kuchens,
   - Zerkleinern des Kuchens, um eine durchschnittliche Partikelgröße von kleiner oder gleich 5 mm zu erhalten,
   - Extrahieren des Rapskuchens mit Ethanol bei 96°,
   - Filtern,
   - Zurückgewinnen des Filtrats,
   - Verdampfen des Lösungsmittels,
   - Zurückgewinnen des gewonnenen Extrats.

8. Verfahren nach vorhergehendem Anspruch, wobei bei der Extraktion das Verhältnis von Rapskuchenmasse zu Ethanolmasse zwischen 1/1 und 1/4 und vorzugsweise bei etwa 1/2 liegt.

9. Verfahren nach Anspruch 7 oder 8, **dadurch gekennzeichnet, dass** die Extraktion über eine Dauer von 1,5 bis 4 Stunden bei einer Temperatur von 12 bis 45 °C durchgeführt wird.

10. Nichttherapeutische, kosmetische oder Lebensmittelzusammensetzung, **dadurch gekennzeichnet, dass** sie wenigstens einen Extrakt nach einem der Ansprüche 1 bis 6 oder hergestellt nach dem Verfahren nach einem der

EP 4 151 203 B1

Ansprüche 7 bis 9, vorzugsweise mit einem Gehalt von 0,001 bis 30 Gew.-% und vorzugsweise von 0,1 bis 20 Gew.-% bezogen auf das Gesamtgewicht der Zusammensetzung umfasst.

11. Kosmetische, nichttherapeutische Zusammensetzung nach vorhergehendem Anspruch, die in einem kosmetisch akzeptablen Medium wenigstens ein Kosmetikum umfasst, das unter den Benetzungsmitteln, den Verdickungsmitteln, den Texturmitteln, den Emulgatoren, den Dispergiermittel, den Schaummittel, den Weichmachern, den Konservierungsmitteln, den Farbstoffen, den Pflanzenextrakten, den Pflanzenfasern, den Mineralien, den pH-korrigierende Wirkstoffen, den Wirkstoffen und den Duftstoffen ausgewählt ist.

12. Kosmetische Zusammensetzung nach einem der Ansprüche 10 oder 11, **dadurch gekennzeichnet, dass** sie zum Auftragen auf die Haut bestimmt ist.

13. Kosmetische, nichttherapeutische Verwendung von wenigstens einem Extrakt nach einem der Ansprüche 1 bis 6 oder hergestellt nach dem Verfahren nach einem der Ansprüche 7 bis 9, oder von wenigstens einer kosmetischen Zusammensetzung nach einem der Ansprüche 10 bis 12 als pigmentierender, selbstbräunender Wirkstoff.

14. Kosmetische, nichttherapeutische Verwendung von wenigstens einem Extrakt nach einem der Ansprüche 1 bis 6 oder hergestellt nach dem Verfahren nach einem der Ansprüche 7 bis 9, oder von wenigstens einer kosmetischen Zusammensetzung nach einem der Ansprüche 10 bis 12 als schlankmachender Wirkstoff insbesondere zur Straffung der Haut der Gesichtskontur.

15. Verfahren zur kosmetischen nichttherapeutischen Behandlung der Haut, umfassend wenigstens einen Schritt des Auftragens auf die Haut von wenigstens einem Extrakt nach einem der Ansprüche 1 bis 6 oder hergestellt nach dem Verfahren nach einem der Ansprüche 7 bis 9 oder von wenigstens einer kosmetischen Zusammensetzung nach einem der Ansprüche 10 bis 12.

**Claims**

1. Rapeseed meal extract **characterized in that** it is obtainable by a preparation method comprising at least the following steps, in this order:

   - providing rapeseed,
   - pressing the rapeseed and recovering the rapeseed meal,
   - grinding the rapeseed meal to obtain an average particle size less than or equal to 5 mm,
   - extracting the rapeseed meal using 96° ethanol,
   - filtering,
   - recovering the filtrate,
   - evaporating the solvent,
   - recovering the extract obtained,

   said rapeseed meal extract comprising from 0.17% to 0.33% by weight of gamma-tocopherols relative to the total weight of the extract and from 0.14% to 0.28% by weight of alpha-tocopherols relative to the total weight of the extract.

2. Rapeseed meal extract according to claim 1, **characterized in that** it comprises from 1 to 5% preferably 2% by weight of proteins relative to the total weight of the extract.

3. Rapeseed meal extract according to any one of the preceding claims, **characterized in that** it has a polyphenol content ranging from 100 to 150 ppm, and preferably 135 ppm.

4. Rapeseed meal extract according to any one of the preceding claims, **characterized in that** it comprises from 50 to 75% by weight of rape oil and from 25 to 50% by weight of volatile compounds, relative to the total weight of the extract.

5. Rapeseed meal extract according to any one of the preceding claims **characterized in that** it comprises from 0.5 to 1.25%, preferably 0.9% by weight of compounds selected from ceramides and $C_s$-$C_{24}$ fatty amides relative to the total weight of the extract.

6. Rapeseed meal extract according to any one of the preceding claims **characterized in that** it comprises 0.25% by

19

weight of gamma-tocopherols relative to the total weight of the extract and 0.20% by weight of alpha-tocopherols relative to the total weight of the extract.

7.  Method for preparing the extract according to any one of the preceding claims comprising at least the following steps, in this order:

    - providing rapeseed,
    - pressing the rapeseed and recovering the rapeseed meal,
    - grinding the rapeseed meal to obtain an average particle size less than or equal to 5 mm,
    - extracting the rapeseed meal using 96° ethanol,
    - filtering,
    - recovering the filtrate,
    - evaporating the solvent,
    - recovering the extract obtained.

8.  Method according to the preceding claim wherein, during the extraction, the mass of rapeseed meal /mass of ethanol ratio is between 1/1 and 1/4 and preferably about 1/2.

9.  Method according to claim 7 or 8, **characterized in that** the extraction is carried out for a duration ranging from 1.5 to 4 hours, at a temperature ranging from 12 to 45°C.

10. Cosmetic, non-therapeutic or food composition,
    **characterized in that** it comprises at least one extract according to any one of claims 1 to 6 or prepared in accordance with the method according to any one of claims 7 to 9, preferably at a content ranging from 0.001 to 30% by weight and preferably from 0.1 to 20% by weight relative to the total weight of the composition.

11. Cosmetic, non-therapeutic composition according to the preceding claim comprising, in a cosmetically acceptable medium, at least one cosmetic agent selected from moistening agents, thickeners, texturing agents, emulsifiers, dispersing agents, foaming agents, emollients, preservatives, colorants, plant extracts, vegetable fibers, minerals, pH adjusting agents, active ingredients and fragrances.

12. Cosmetic composition according to any one of claims 10 or 11, **characterized in that** it is intended to be applied to the skin.

13. Cosmetic, non-therapeutic use of at least one extract according to any one of claims 1 to 6 or prepared in accordance with the method according to any one of claims 7 to 9, or of at least one cosmetic composition according to any one of claims 10 to 12 as a pro-pigmenting, self-tanning active ingredient.

14. Cosmetic, non-therapeutic use of at least one extract according to any one of claims 1 to 6 or prepared in accordance with the method according to any one of claims 7 to 9, or of at least one cosmetic composition according to any one of claims 10 to 12 as a slimming active ingredient notably for firming the skin of the facial contour.

15. Method of cosmetic, non-therapeutic skin treatment, comprising at least one step of applying to the skin, at least one extract according to any one of claims 1 to 6 or prepared in accordance with the method according to any one of claims 7 to 9, or of at least one cosmetic composition according to any one of claims 10 to 12.

[Fig.1]

[Fig.2]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

• FR 2904555 A1

**Littérature non-brevet citée dans la description**

• **YANG T.** ; **SINAI P.** ; **KAIN S.** An acid phosphatase assay for quantifying the growth of adherent and nonadherent cells.. *Anal. Biochem.*, 1996, vol. 24, 103-108